(19) Europäisches Patentamt / European Patent Office / Office européen des brevets

(11) **EP 3 735 952 A1**

(12) **EUROPEAN PATENT APPLICATION**
published in accordance with Art. 153(4) EPC

(43) Date of publication:
**11.11.2020 Bulletin 2020/46**

(21) Application number: **18898959.4**

(22) Date of filing: **27.12.2018**

(51) Int Cl.:
**A61H 9/00** *(2006.01)*    **A61H 19/00** *(2006.01)*

(86) International application number:
**PCT/KR2018/016729**

(87) International publication number:
**WO 2019/135547 (11.07.2019 Gazette 2019/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.01.2018 KR 20180000829
03.01.2018 KR 20180000828**

(71) Applicant: **CG Bio Co., Ltd.
Seongnam-si, Gyeonggi-do 13211 (KR)**

(72) Inventors:
• **KIM, In Ho
Gyeonggi-do 17809 (KR)**

• **JO, Young Yoon
Seoul 06708 (KR)**
• **YOO, Bo Young
Seoul 06708 (KR)**
• **KU, Ja Hong
Pyeongtaek-si Gyeonggi-do 17941 (KR)**
• **KANG, Gi Hun
Seongnam-si Gyeonggi-do 13228 (KR)**
• **KIM, Saes Byul
Seoul 08803 (KR)**

(74) Representative: **Eisenführ Speiser
Patentanwälte Rechtsanwälte PartGmbB
Postfach 10 60 78
28060 Bremen (DE)**

(54) **CANISTER, SUCTION APPARATUS AND TISSUE EXPANSION APPARATUS COMPRISING SAME**

(57)    The present invention relates to a canister, a suction apparatus and a tissue expansion apparatus comprising same, and provides, according to one aspect of the present invention, a suction apparatus comprising: a housing having an inlet and an outlet; a pump disposed in the housing and suctioning air flowing in via the inlet; a first flow line for connecting the inlet and an inflow port of the pump; a second flow line for connecting an outflow port of the pump and outside air; a control valve disposed in the housing and provided with a first port connected to the first flow line, and a second port connected to the second flow line; a battery disposed in the housing and supplying power to the pump; and a control unit for controlling the pump and the control valve.

[FIGURE 12]

500
560
550
540
532 533
530
521
520
510

EP 3 735 952 A1

**Description**

[Technical Field]

The present invention relates to a dome for tissue expansion, a canister, a suction apparatus, and a tissue expansion apparatus including the same and a breast volume measuring method using the same.

[Background Art]

A plastic surgery field, in which congenital malformations and acquired injuries are treated to recover intrinsic functions of body parts or to achieve normal appearances through a surgical operation, has recently developed into general cosmetic surgery.
Particularly, in general cosmetic surgery, a plastic surgery field has recently been expanded to fields such as breast augmentation/reduction, liposuction, penis enlargement, and the like in addition to facial plastic surgery.
Particularly, for breast augmentation plastic surgery, penis enlargement plastic surgery, and the like, a surgical operation that inserts prostheses such as silicone is performed. However, cases of various side effects caused by prostheses such as silicone have been reported.
Accordingly, apparatuses and methods for augmenting female breasts in a manner other than invasive surgery have been proposed.

[Disclosure]

[Technical Problem]

The present invention is directed to providing a dome for tissue expansion, a canister, a suction apparatus, and a tissue expansion apparatus including the same and a breast volume measuring method using the same.

[Technical Solution]

One aspect of the present invention provides a suction apparatus including a housing having an inlet and an outlet, a pump disposed in the housing and configured to suction air introduced through the inlet, a first flow line connecting the inlet to an inflow port of the pump, a second flow line connecting an outflow port of the pump to the external atmosphere, a control valve disposed in the housing and having a first port connected to the first flow line and a second port connected to the second flow line, a battery disposed in the housing and configured to supply power to the pump, and a control unit configured to control each of the pump and the control valve.
The suction apparatus may further include an air distribution member mounted on the pump and having an inlet passage forming at least a portion of the first flow line and connected to the inflow port of the pump and an outlet passage forming at least a portion of the second flow line and connected to the outflow port of the pump.
The control valve may be mounted on the air distribution member such that the first port is connected to the inlet passage and the second port is connected to the outlet passage.
When the pump operates and the control valve is closed, air may be suctioned into the pump along the inlet passage of the air distribution member through the inlet, and the air may be discharged to the outside along the outlet passage.
When the pump is stopped and the control valve is open, outside air may flow into the second port of the control valve along the outlet passage, may pass through the first port, and may be movable to the inlet along the inlet passage.
A flow hole may be provided in the inlet passage of the air distribution member, and a check valve, for allowing a flow only in a direction toward the inflow port of the pump, may be provided at the flow hole.
The check valve may open the inlet passage toward the inflow port of the pump when the pump operates and close the inlet passage when the pump is stopped.
The check valve may include a shaft inserted into the flow hole, a sealing member mounted on the shaft and coming into contact with the flow hole, and an elastic member provided to be compressed when the pump operates and thus the shaft moves toward the pump.
When the shaft moves toward the pump, the sealing member may be separated from the flow hole.
The suction apparatus may further include a discharge pipe connecting an outlet passage of an air distribution member to the outlet of the housing and forming at least a portion of the second flow line.
The suction apparatus may further include a negative pressure sensing unit which is disposed in the housing, has a flow path connecting the inlet to an inlet passage of an air distribution member, and is configured to measure a pressure of the air suctioned through the inlet.

The suction apparatus may further include a canister mounted on a side of the inlet of the housing.

Another aspect of the present invention provides a tissue expansion apparatus including a dome for tissue expansion and a suction apparatus detachably mounted to the dome.

The dome may include a cap including a receiving part covering a partial region of a wearer's body, a suction port connected to the receiving part such that a fluid is movable therebetween, and a mounting part surrounding the suction port, and a contact part provided along an edge of the cap to come into contact with the wearer's body.

The suction apparatus may include a housing that has an inlet connected to the suction port and an outlet, and is accommodated in the mounting part, a pump disposed in the housing and configured to suction air introduced through the inlet, a first flow line connecting the inlet to an inflow port of the pump, a second flow line connecting an outflow port of the pump to the external atmosphere, a control valve disposed in the housing and having a first port connected to the first flow line and a second port connected to the second flow line, a battery disposed in the housing and configured to supply power to the pump, and a control unit configured to control each of the pump and the control valve.

At least one magnet may be provided in each of the mounting part of the cap and the housing.

The tissue expansion apparatus may further include a canister mounted on a side of the inlet of the housing.

The canister may include a suctioning port connected to an interior of the dome, a discharge port connected to the inlet, and a flow path connecting the suctioning port to the discharge port.

The flow path may include a plurality of first sections provided such that air flows parallel to a central axis direction of the suctioning port and second sections prepared to connect two neighboring first sections such that air flows in a radial direction based on a central axis of the suctioning port.

Still another aspect of the present invention provides a tissue expansion apparatus including a dome configured to receive a wearer's body tissue and a suction apparatus mounted on the dome to generate negative pressure within the dome. The suction apparatus includes a housing having an inlet connected to an interior of the dome and an outlet, a pump disposed in the housing and configured to suction air inside the dome through the inlet, and a canister mounted on a side of the inlet of the housing. The canister includes a suctioning port connected to the interior of the dome, a discharge port connected to the inlet, and a flow path connecting the suctioning port to the discharge port, and the flow path includes a plurality of first sections prepared such that air flows in parallel to a central axis direction of the suctioning port and second sections prepared to connect two neighboring first sections such that air flows in a radial direction based on a central axis of the suctioning port.

The second sections may be prepared such that air flows in a certain rotating direction based on the central axis of the suctioning port.

The suctioning port and the discharge port may be prepared to be located coaxially.

The canister may include a suction member formed of an elastic member and forming the suctioning port, a lower case on which the suction member is mounted, an upper case provided with the discharge port and mounted on the lower case to form a predetermined flow space, a partition member disposed in the flow space and configured to divide the flow space into the plurality of first sections and second sections, and a filter disposed between the partition member and the upper case.

The lower case may include a plurality of first flow guides, which have a ring shape and different diameters and are arranged concentrically, on a first surface thereof facing the upper case.

The upper case may include a second flow guide, which is located between two neighboring first flow guides, on a first surface thereof facing the upper case.

The first flow guide may have a height in which the first flow guide does not come into contact with the upper case, and the second flow guide may have a height in which the second flow guide does not come into contact with the lower case.

A space between the first flow guide and the second flow guide may form the first section, and a space between the second flow guide and the first surface of the lower case may form the second section.

A plurality of spacers may be provided on a second surface of the upper case, which is a surface opposite to the first surface, to support the filter.

A space between a second surface of the upper case and the filter may be connected to the discharge port.

Super absorbent polymer particles may be disposed in the flow space.

The canister may include a sealing member mounted on a side of the discharge port of the upper case and coming into contact with a side of the inlet of the housing.

Yet another aspect of the present invention provides a tissue expansion apparatus including a housing having an inlet and an outlet, a pump disposed in the housing and configured to suction air introduced through the inlet, and a canister mounted on a side of the inlet of the housing. The canister includes a suction member formed of an elastic member and forming the suctioning port, a lower case on which the suction member is mounted, an upper case provided with a discharge port and mounted on the lower case to form a predetermined flow space, a partition member disposed in the flow space and configured to divide the flow space into a plurality of flow spaces, and a filter disposed between the partition member and the upper case. A flow path in the flow space may include a plurality of first sections prepared such that air flows in parallel to a central axis direction of the suctioning port and second sections prepared to connect two neighboring first sections such that air flows in a radial direction based on a central axis of the suctioning port.

[Advantageous Effects]

As described above, a dome for tissue expansion, a canister, a suction apparatus, and a tissue expansion apparatus including the same in accordance with one embodiment of the present invention have effects as follows.

A dome can be stably adhered closely to a wearer's body tissue (for example, a breast) by providing various form factors that are suitable for a wearer's physical features to a contact region of the dome, thereby effectively generating negative pressure within the dome.

Further, by providing a structure in which a suction apparatus is detachably mounted on a dome, a wearer's convenience in use can be improved, and since the suction apparatus is mounted on the dome, it is possible to shorten the physical distance between a negative pressure source and a negative pressure generation space.

Further, a change in breast volume can be measured on the basis of a flow rate of air flowing into a dome. In addition, an apparatus for measuring a breast volume can be provided separately from a suction apparatus, and the apparatus can be detachably mounted on the suction apparatus when necessary.

Further, moisture can be prevented from being generated in a canister in a suction apparatus during operation.

[Description of Drawings]

FIG. 1 is a schematic view illustrating a tissue expansion apparatus in accordance with one embodiment of the present invention.

FIG. 2 is a perspective view illustrating a tissue expansion apparatus in accordance with one embodiment of the present invention.

FIG. 3 is a plan view illustrating a dome for tissue expansion in accordance with one embodiment of the present invention.

FIGS. 4 and 5 are views illustrating an air tube constituting the dome for tissue expansion in accordance with one embodiment of the present invention.

FIG. 6 is a perspective view illustrating a state in which a partial region of the air tube is cut off.

FIG. 7 is a partially enlarged view of FIG. 6.

FIG. 8 is an exploded perspective view illustrating the tissue expansion apparatus in accordance with one embodiment of the present invention.

FIG. 9 is a perspective view illustrating a suction apparatus in accordance with one embodiment of the present invention.

FIGS. 10 and 11 are block diagrams illustrating an operating state of the suction apparatus in accordance with one embodiment of the present invention.

FIG. 12 is an exploded perspective view of the suction apparatus in accordance with one embodiment of the present invention.

FIGS. 13 and 14 are perspective views illustrating some elements of the suction apparatus shown in FIG. 12.

FIG. 15 is a view for describing an operating state of an air distribution member and a pump.

FIG. 16 is a perspective view illustrating some elements of the suction apparatus shown in FIG. 12.

FIG. 17 is a perspective view of the suction apparatus in accordance with one embodiment of the present invention.

FIG. 18 is a negative pressure graph for describing an operating state of the suction apparatus in accordance with one embodiment of the present invention.

FIGS. 19 to 23 are block diagrams for describing a volume measuring apparatus.

FIG. 24 is a schematic view illustrating a tissue expansion apparatus according to another embodiment.

FIGS. 25 and 26 are exploded perspective views of a canister constituting the suction apparatus in accordance with one embodiment of the present invention.

FIG. 27 is a cross-sectional view of the state in which all elements shown in FIG. 25 are assembled.

[Modes of the Invention]

Hereinafter, a dome for tissue expansion, a canister, a suction apparatus, and a tissue expansion apparatus including the same according to one embodiment of the present invention will be described in detail with reference to the accompanying drawings.

Further, regardless of the reference numerals, identical or corresponding elements are denoted by the same or similar reference numerals, and duplicate descriptions thereof will be omitted. In addition, for convenience of description, the size and shape of each element illustrated in the drawings may be exaggerated or reduced.

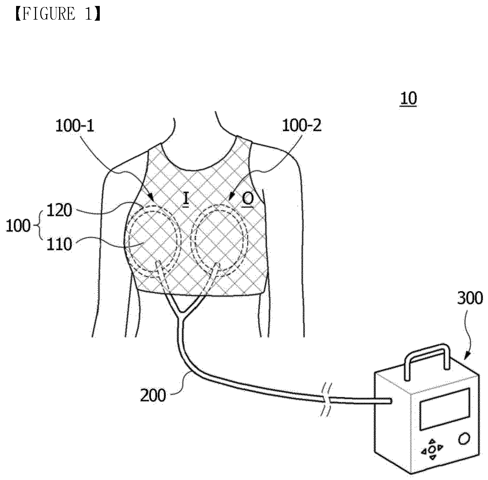

FIG. 1 is a schematic view illustrating a tissue expansion apparatus 10 in accordance with one embodiment of the present invention.

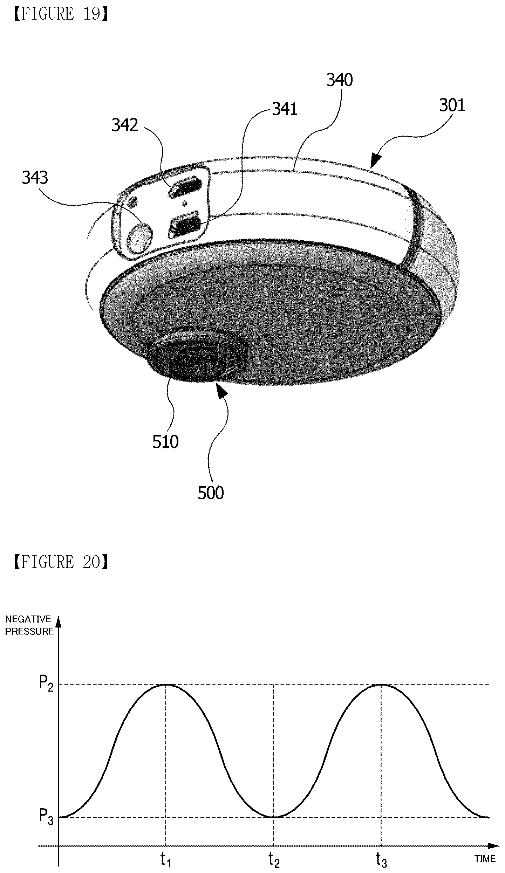

The tissue expansion apparatus 10 in accordance with one embodiment of the present invention includes domes 100 (100-1 and 100-2) for tissue expansion configured to receive a partial region of a wearer's body, and a suction apparatus 300 configured to generate negative pressure within an inner space 113 (a receiving part, see FIG. 19) of the dome 100. Further, the inner space of the dome 100 may be connected to the suction apparatus 300 through a connection tube 200. The domes 100 may be provided in a pair at wearer's left and right breasts, and, in the present invention, a right breast dome 100-1 and a left breast dome 100-2 may have left and right symmetrical shapes. Hereinafter, the domes 100 for tissue expansion will be described taking the right breast dome 100-1 as an example. In addition, in FIG. 1, a reference numeral "I" represents an inner region of the breast, and a reference numeral "O" represents an outer region of the breast.

In the following description, it will be understood that the terms "a wearer's body tissue" or "a partial region of a wearer's body" may mean an arbitrary partial region of the wearer's body requiring tissue expansion, and for example, the wearer's body tissue may be a wearer's breast.

The dome 100 includes a cap 110 surrounding a partial region of the wearer's body, for example, a breast, and a contact part provided at an edge of the cap 110 to be closely adhered to the wearer's body region.

The cap 110 may have a shape open toward the wearer's body region, for example, a hemispherical shape. The cap 110 has a peak 111 which is located at the maximum height in a state in which the cap 110 comes into contact with the wearer's body.

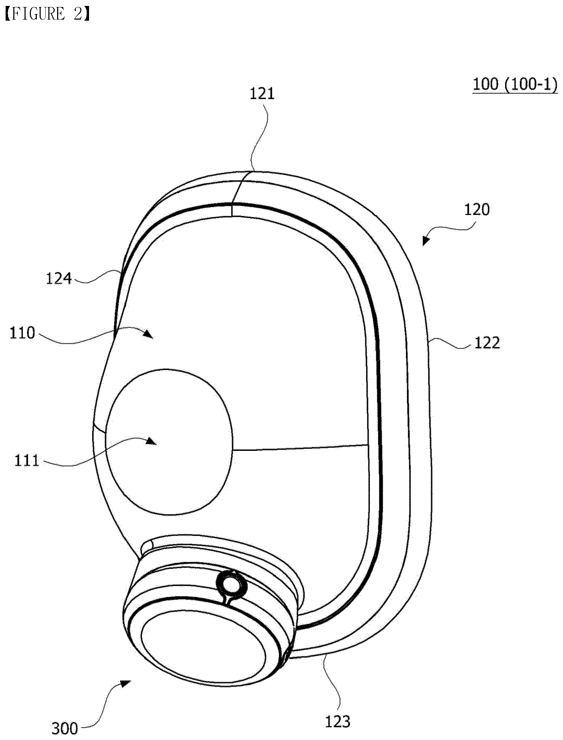

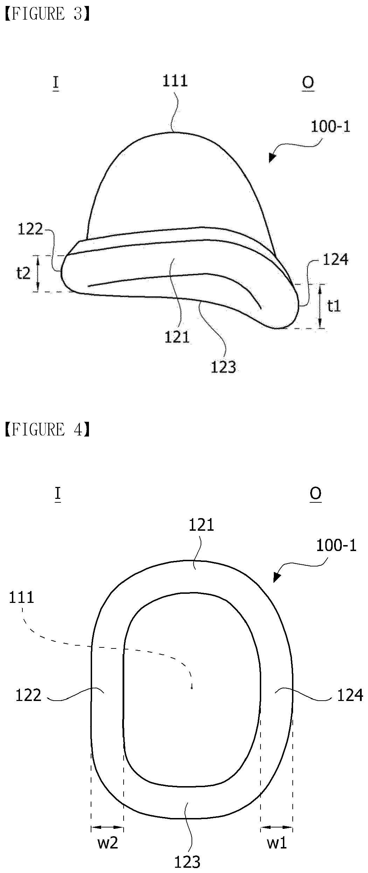

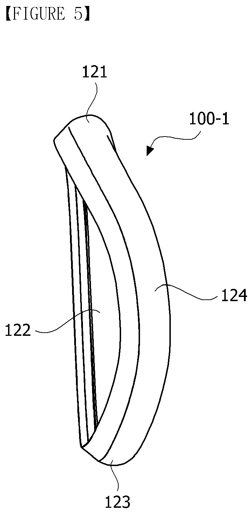

FIG. 2 is a perspective view illustrating a tissue expansion apparatus in accordance with one embodiment of the present invention, FIG. 3 is a plan view illustrating a dome 100-1 for tissue expansion in accordance with one embodiment of the present invention, and FIGS. 4 and 5 are views illustrating an air tube 120 constituting the dome 100-1 for tissue expansion in accordance with one embodiment of the present invention.

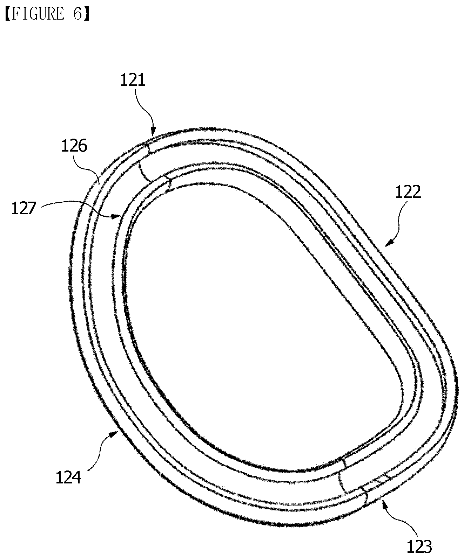

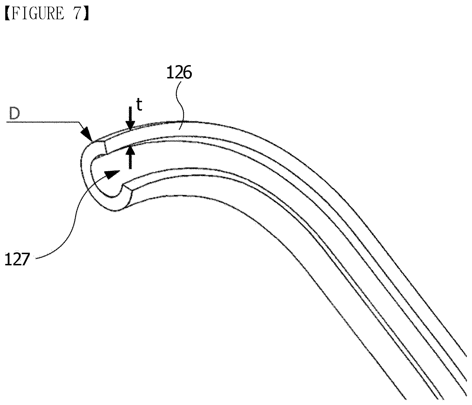

Further, FIG. 6 is a perspective view illustrating a state in which a partial region of the air tube 120 is cut off, and FIG. 7 is a partially enlarged view of FIG. 6.

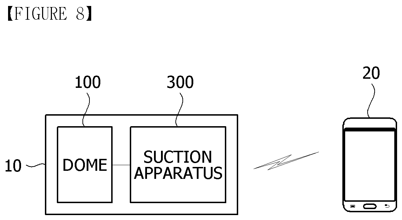

Referring to FIG. 2, the dome 100-1 for tissue expansion includes a cap 110 having a receiving part 113 covering a partial region of a wearer's body, and a suction port 112 (see FIG. 8) connected to the receiving part 113 such that a fluid is movable therebetween. The cap 110 may be formed of various resin materials, for example, at least one of a polycarbonate (PC) resin and a polyethylene (PE) resin.

Further, the dome 100-1 may include an air tube 120 provided along an edge of the cap 110 so as to come into contact with the wearer's body and having an air-fillable interior.

In the present embodiment, the contact part of the dome 100-1 is the air tube 120. The air tube may have negative pressure buffering effects and an effect of improving adhesion of the dome. The air tube 120 may be formed of various materials, for example, at least one of a polyvinyl chloride (PVC) resin and a polyurethane (PU) resin.

The air tube 120 may have a diameter D which is varied in at least some regions along the edge of the cap based on the peak 111 of the cap 110.

Further, the air tube 120 may include, based on the peak 111 of the cap 110, a first region 121 located at an upper region of the edge of the cap 110, a second region 122 located at a right region of the edge of the cap 110, a third region 123 located at a lower region of the edge of the cap 110, and a fourth region 124 located at a left region of the edge of the cap 110. Referring to FIGS. 1 and 3, the second region 122 is closely adhered to the inner region of the breast (a central cleavage region), and the fourth region is closely adhered to the outer region of the breast.

Here, the second region and the fourth region have shapes which are bilaterally asymmetrical to each other about the peak 111 of the cap 110. The asymmetrical shape is provided in consideration of properties of body tissues around the breast and is configured to improve adhesion to the breast and apply uniform negative pressure over the entire breast when the negative pressure is provided.

Further, the air tube 120 may be provided such that a distance between the second region 122 and the peak 111 and a distance between the fourth region 124 and the peak 111 are different. Here, one region of the second region 122 and the fourth region 124, which has a longer distance from the peak 111, may be bent to have a predetermined curvature.

For example, in the case of the right breast dome 100-1, the second region 122 may be a region having a shorter distance from the peak 111 and the fourth region 124 may be a region having a longer distance from the peak 111. Here, the fourth region 124 may be bent to have a predetermined curvature. That is, the fourth region 124 located at the outer region O of the breast may have a longer distance from the peak than the second region 122 located at the inner region I of the breast and may be bent to have a predetermined curvature.

Referring to FIG. 4, among the second region 122 and the fourth region 124, at least a portion of one region (the second region 122 in the case of the right breast dome) having a shorter distance from the peak 111 may be formed in a rectilinear shape. In the case that the second region 122 is formed in a rectilinear shape, when the wearer wears the dome 100-1, the second region 122 of the dome 100-1 may be easily aligned with central cleavage of the wearer.

Referring to FIGS. 3 and 4, among the second region 122 and the fourth region 124, the region (the fourth region 124 in the case of the right breast dome) having a longer distance from the peak 111 may be formed to have a diameter which is 1.5 to 2.5 times a diameter of the region (the second region 122 in the case of the right breast dome) having a shorter distance from the peak 111.

Referring to FIG. 3, among the second region 122 and the fourth region 124, the region having a longer distance from the peak 111 (the fourth region 124 in the case of the right breast dome) may be formed to have a thickness which is 1.5 to 2.5 times a thickness of the region (the second region 122 in the case of the right breast dome) having a shorter distance from the peak 111 (t1>t2).

Further, referring to FIG. 4, among the second region 122 and the fourth region 124, the region (the fourth region 124 in the case of the right breast dome) having a longer distance from the peak 111 may be formed to have a width which is 1.5 to 2.5 times a width of the region (the second region 122 in the case of the right breast dome) having a shorter distance from the peak 111 (w1>w2).

Referring to FIG. 5, among the second region 122 and the fourth region 124, the region (the fourth region 124 in the case of the right breast dome) having a longer distance from the peak 111 protrudes toward the wearer's body more than the region (the second region 122 in the case of the right breast dome) having a shorter distance from the peak 111.

Further, the first region 121 and the third region 123 may be bent to have different curvatures, and the curvature of the first region 121 may be greater than the curvature of the third region 123. Such a design having a curvature difference may correspond to a difference in curvatures between upper and lower regions of the wearer's breast.

Further, among the second region 122 and the fourth region 124, the curvature of the region (the fourth region 124 in the case of the right breast dome) having a longer distance from the peak 111 may be greater than the curvature of the first region.

Referring to FIGS. 6 and 7, the air tube 120 may include a space part 127 into which air is injected and a film 126 that is expandable and defines the space part 127.

Here, at least one of a diameter of the space part 127 and a thickness t of the film 126 of the air tube 120 may be varied in at least some regions along the edge of the cap 110 based on the peak 111 of the cap 110.

As described above, since at least one of the diameter of the space part 127 and the thickness t of the film 126 is varied, the diameter D of the air tube may be varied as described above with reference to FIGS. 3 to 5.

For example, among the second region 122 and the fourth region 124, the region having a longer distance from the peak 111 (the fourth region 124 in the case of the right breast dome) may have a diameter of the injection space part and a thickness of the film which are greater than those of the region (the second region 122 in the case of the right breast dome) having a shorter distance from the peak 111.

In contrast, the diameter D of the air tube 120 may be varied according to the thickness of the film 126 in a state in which the space part 127 has the same diameter.

Further, the air tube 120 may be provided to be expanded according to the injection of air, and a volume of the air injected into the air tube 120 may be 60 to 80% of the maximum air capacity thereof. By injecting a smaller volume of air than the maximum air capacity, adhesion and negative pressure buffering effects may be improved.

Further, in order to improve adhesion, an embossed or engraved pattern to increase a contact area, such as convex and concave shapes, may be formed on a surface of the film 126.

Further, in order to improve adhesion, an adhesive layer having excellent biocompatibility and predetermined adhesive strength may be additionally formed on the surface of the film 126.

FIG. 8 is an exploded perspective view illustrating the tissue expansion apparatus in accordance with one embodiment of the present invention.

The tissue expansion apparatus includes a dome 100 for tissue expansion and a suction apparatus 300 configured to generate negative pressure within the dome 100.

As described above, the dome 100 for tissue expansion may include a cap 110 having a receiving part 113 covering a partial region of a wearer's body, and a suction port 112 connected to the receiving part 113 such that a fluid is movable therebetween and connected to the suction apparatus 300, and an air tube 120 provided along an edge of the cap 110 so as to come into contact with the wearer's body and having an air-fillable interior. Further, a diameter of the air tube 120 may be varied in at least some regions along the edge of the cap 110 based on a peak 111 of the cap 110.

Meanwhile, the cap 110 may have a mounting part 130 so that the suction apparatus 300 is detachably mounted thereto. For example, the mounting part 130 may be a ring-shaped mounting rib 131. Here, the above-described suction port 112 may be provided to be located in the mounting part 130.

The cap 110 may be coupled to the suction apparatus 300 by magnetic force using magnets. To this end, one or more magnets may be provided on each of the mounting part 130 of the cap 110 and the suction apparatus 300.

Further, the mounting part may be at least partially recessed toward an interior of the cap. Due to such a structure, the suction apparatus 300 may be in a state of being partially recessed toward the interior of the cap 110, and thus a region of the suction apparatus 300 exposed to an exterior of the cap 110 may be reduced so that the overall volume of the tissue expansion apparatus may be reduced.

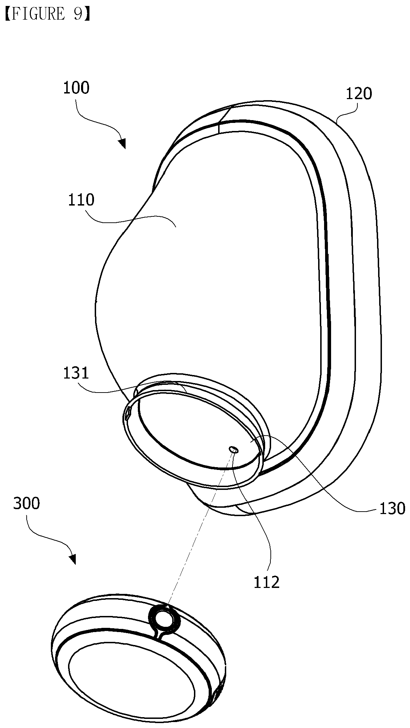

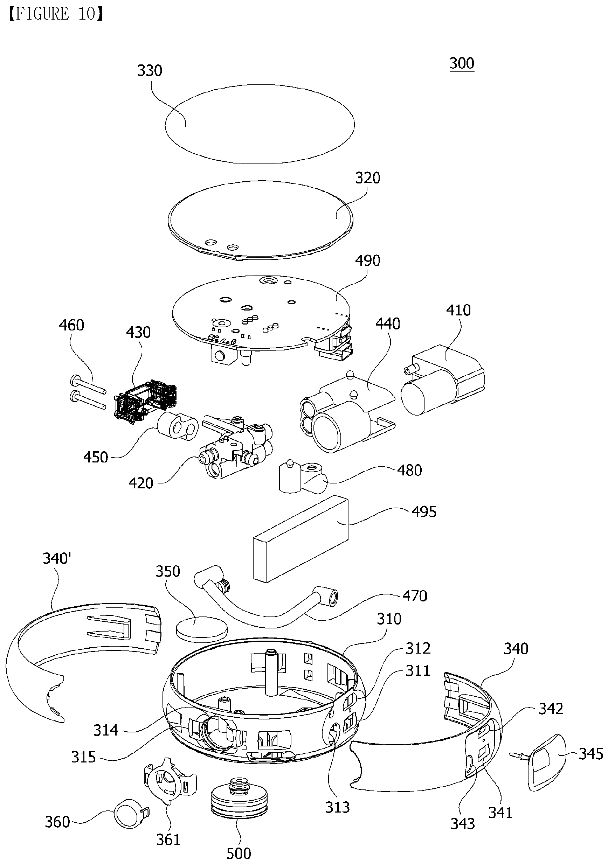

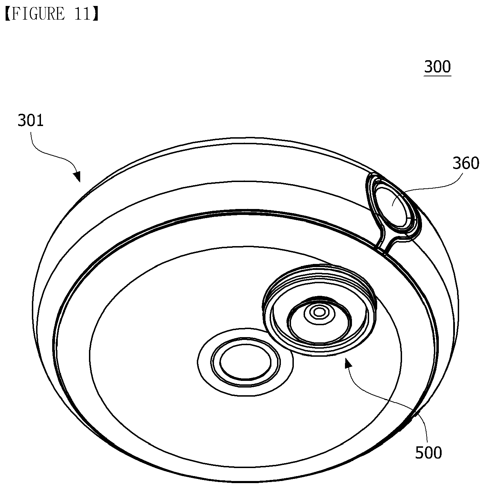

FIG. 9 is a perspective view illustrating the suction apparatus 300 in accordance with one embodiment of the present invention, and FIGS. 10 and 11 are block diagrams illustrating an operating state of the suction apparatus in accordance with one embodiment of the present invention.

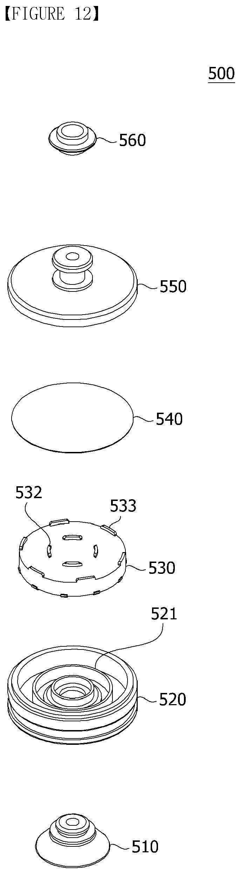

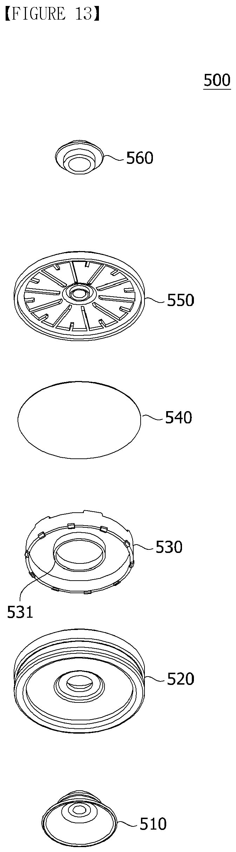

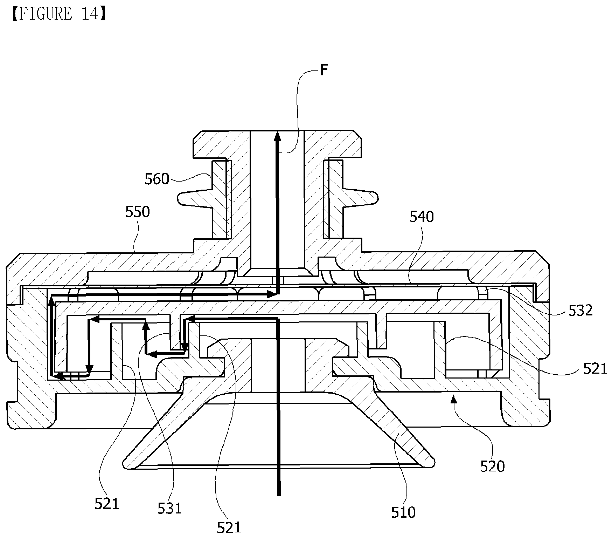

Further, FIG. 12 is an exploded perspective view of the suction apparatus 300 in accordance with one embodiment of the present invention, and FIGS. 13 and 14 are perspective views illustrating some elements of the suction apparatus shown in FIG. 12.

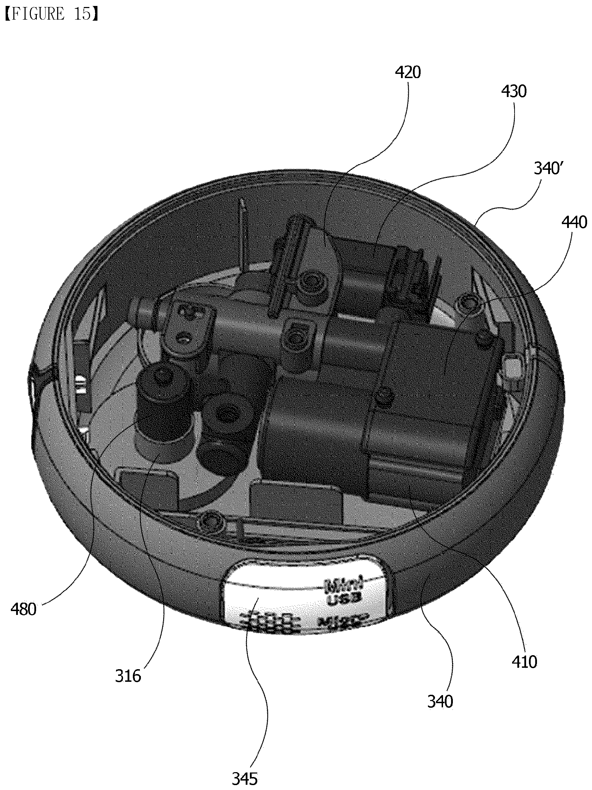

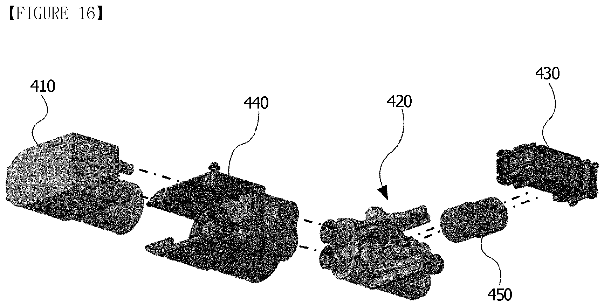

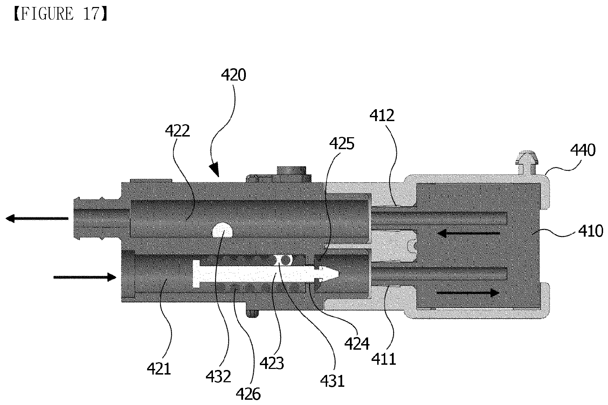

Further, FIG. 15 is a view for describing an operating state of an air distribution member and a pump, FIG. 16 is a perspective view illustrating some elements of the suction apparatus shown in FIG. 12, and FIG. 17 is a perspective view of the suction apparatus in accordance with one embodiment of the present invention.

The suction apparatus 300 in accordance with one embodiment of the present invention includes a housing 301 having an inlet 302 and an outlet 303. The inlet 302 is connected to the above-describe suction port 112 of the cap 110 such that a fluid is movable therebetween. In such a structure, air inside the cap 110 is suctioned into the suction apparatus 300 via the inlet 302 through the suction port 112. Meanwhile, a canister 500 may be disposed at the inlet 302, and in the process of air flowing, the inlet 302 of the housing 301 may mean an inlet of the canister 500.

Further, the suction apparatus 300 includes a pump 410 disposed within the housing 301 and configured to suction air introduced through the inlet 302.

Further, the suction apparatus 300 includes a first flow line 701 connecting the inlet 302 to an inflow port 411 of the pump 410 and a second flow line 702 connecting an outflow port 412 of the pump 410 to the external atmosphere. Here, the second flow line 702 is open to the outside and thus connects the outflow port 412 of the pump 410 to the external atmosphere.

Further, the suction apparatus 300 includes a control valve 430 disposed in the housing 301 and having a first port 431 connected to the first flow line and a second port 432 connected to the second flow line. The control valve 430 has a passage connecting the first port 431 to the second port 432 and is provided to open and close the passage.

Meanwhile, an undescribed reference numeral 703 represents a third flow line connecting the first flow line 701 to the first port 431 of the control valve 430, and an undescribed reference numeral 704 represents a fourth flow line connecting the second flow line 702 to the second port 432 of the control valve 430.

Further, the suction apparatus 300 includes a battery 495 disposed in the housing 301 and configured to supply power to the pump 410, and the suction apparatus 300 includes a control unit 490 (also referred to as a "first control unit" in the following description) configured to respectively control the pump 410 and the control valve 430.

Referring to FIG. 15, the suction apparatus 300 may further include an air distribution member 420 mounted on the pump 410. The air distribution member 420 has an inlet passage 421 forming at least a portion of the first flow line 701 and connected to the inflow port 411 of the pump 410, and an outlet passage 422 forming at least a portion of the second flow line 702 and connected to the outflow port 412 of the pump 410.

Here, the control valve 430 is mounted on the air distribution member 420 such that the first port 431 is connected to the inlet passage 421 and the second port 432 is connected to the outlet passage 422. Meanwhile, the air distribution member 420 is coupled to the control valve 430 through a vibration isolation mount 450 formed of an elastic material, such as rubber. For example, the control valve 420 may be mounted on the air distribution member 420 through a binding member such as a reference numeral 460.

Referring to FIGS. 10 and 15, when the pump 410 operates and the control valve 430 is closed, air is suctioned into the pump 410 along the inlet passage of the air distribution member 421 through the inlet 302, and the air is discharged to the outside along the outlet passage 422. In this case, negative pressure is generated at the interior 113 of the dome 100. In more detail, when the suction apparatus 300 operates in a negative pressure mode, the control unit 490 operates the pump 410 and closes the control valve 430, and air at the interior 113 of the dome 100 is suctioned into the suction apparatus 300, and thus the negative pressure is generated within the dome 100.

Meanwhile, referring to FIGS. 11 and 15, when the pump 410 is stopped and the control valve 430 is open, outside air flows into the outlet 303 of the housing, flows into the second port 432 of the control valve 430 along the outlet line 422, passes through the first port 431, and is movable to the inlet along the inlet passage 421. In this case, since the outside air flows into the interior 113 of the dome 100, the negative pressure within the dome 100 is released based on the introduced air amount.

As described above, the control unit 490 may control the negative pressure within the dome by controlling the operation of the pump 410 and the turning on and off of the control valve 430.

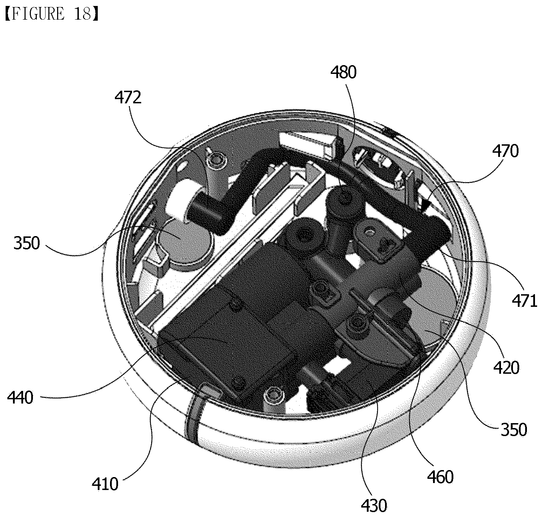

FIG. 18 is a negative pressure graph for describing an operating state of the suction apparatus in accordance with one embodiment of the present invention. For example, as illustrated in FIG. 18, the control unit may perform a cycle mode in which the negative pressure within the dome is continuously changed between two set negative pressure values.

Referring to FIG. 15, a flow hole 424 may be provided in the inlet passage 421 of the air distribution member 420, and a check valve, for allowing a flow only in a direction toward the inflow port 411 of the pump 410, may be provided at the flow hole 424.

In more detail, the check valve operates to open the flow hole 424 so that the inlet passage 421 is opened toward the inflow port 411 of the pump when the pump 410 operates, and the check valve operates to close the flow hole 424 so that the inlet passage 421 is closed when the pump 410 does not operate.

As one example, the check valve may include a shaft 423 inserted into the flow hole 424, a sealing member 425 mounted on the shaft 423 and coming into contact with the flow hole 424, and an elastic member 426 (for example, a spring) provided to be compressed when the pump 410 operates and thus the shaft 423 moves toward the pump 410. Here, when the pump operates and thus the shaft 423 moves toward the pump 410 by the pressure of air flowing into the inlet passage 421, the sealing member 425 is separated from the flow hole 424 to open the flow hole 424.

Further, the suction apparatus 300 additionally includes a discharge pipe 470 connecting the outlet passage 422 of the air distribution member to the outlet 303 of the housing 301 and forming at least a portion of the second flow line 702.

Further, the suction apparatus 300 may additionally include a vibration isolation member 440 mounted to surround the pump 410.

Further, the suction apparatus 300 may additionally include a negative pressure sensing unit 480 (also referred to as a "pressure sensor" in the following description) which is disposed in the housing 301, has a flow path connecting the inlet 302 to the inlet passage 421 of the air distribution member 420, and measures negative pressure at the interior 113 of the dome 100 connected to the inlet 302. As an example, the pressure sensor 480 may be provided to measure negative pressure within the dome by measuring an amount of air in the dome.

The housing 301 includes a main case 310 in which the pump, the air distribution member, the control valve, the discharge pipe, the negative pressure sensing unit 480, the battery 495, and the control unit 490 (a circuit board) are disposed. The main case 310 has a structure in which an upper surface is open and the canister 500 is mounted on a lower surface.

Further, the housing 310 includes an upper case 320 covering the open region of the main case 310. In addition, the housing 310 may additionally include a transparent decorative case 330 mounted on the upper case 320.

Further, the housing 310 includes a plurality of side panels 340 and 340' mounted on a side surface of the main case 310.

Further, the control unit 490 includes a connection terminal electrically connected to a volume measuring apparatus which will be described later, and a charging terminal for charging the battery. Here, the main case 310 includes a first hole 311 and a second hole 312 to respectively expose the connection terminal and the charging terminal to the outside of the housing 301. Further, the main case 310 includes a third hole 313 that performs the function of the outlet 303 of the housing 301.

Further, the side panel 430, which is mounted on the main case 310 to surround the first to third holes 311, 312, and 313, includes first to third exposure holes 341, 342, and 343 configured to respectively expose the first to third holes 311, 312, and 313 to the outside.

Further, a cover member 345 may be detachably mounted on the side panel 340 to surround the first to third exposure holes 341, 342, and 343. However, at least some regions of the cover member 345 may be provided, for example, as a mesh region, so as to expose the third exposure hole 343 corresponding to the third hole 313 that performs the function of the outlet 303 of the housing 301.

Meanwhile, a power switch 360 configured to turn the suction apparatus 300 on and off is mounted on the main case 310, and the power switch 360 may be mounted on the main case 310 through a mounting bracket 361. The main case 310 is provided with a fourth hole 314, which is opened so that the power button 360 is electrically connected to the control unit, and a fifth hole 315 to which the mounting bracket 361 is fixed.

Further, in the present embodiment, the tissue expansion apparatus includes the dome 100 for tissue expansion and the suction apparatus 300 detachably mounted on the dome 100.

Further, as described above, the dome 100 includes the cap 110 including the receiving part 113 covering a partial region of a wearer's body, the suction port 112 connected to the receiving part such that a fluid is movable therebetween, and the mounting part 130 surrounding the suction port 112, and the contact part provided along the edge of the cap 110 to come into contact with the wearer's body.

Meanwhile, one or more magnets 350 may be disposed at each of the mounting part 130 of the cap 110 and the interior of the housing 301 of the suction apparatus 300.

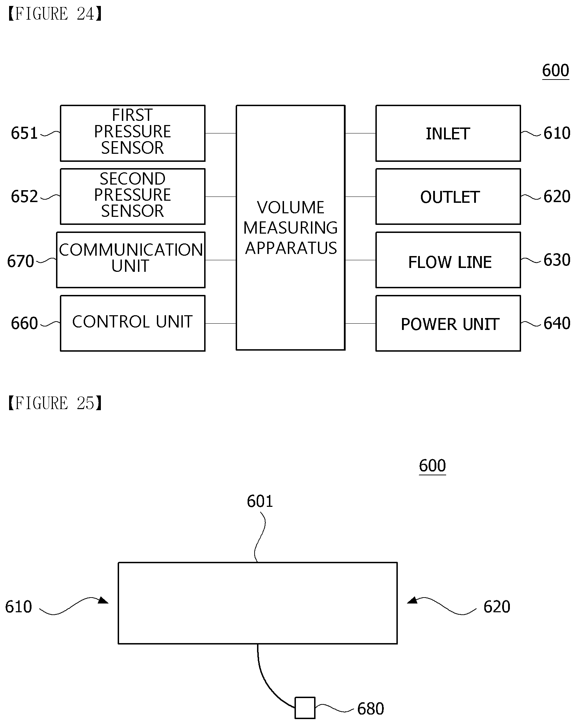

FIGS. 19 to 23 are block diagrams for describing the volume measuring apparatus, and FIG. 24 is a schematic view illustrating a tissue expansion apparatus according to another embodiment.

According to the present embodiment, a breast volume may be measured using the suction apparatus having the above-described structure and the tissue expansion apparatus including the same.

A breast volume measuring method in accordance with one embodiment of the present invention relates to a method of measuring a breast volume using a tissue expansion apparatus 10 including domes 100 for tissue expansion worn on breasts and a suction apparatus 300 including a pump 410 that generates negative pressure within the dome 100.

The breast volume measuring method includes a first operation of stopping the operation of the pump 410 when an interior 113 of the dome 100 has a first set pressure and allowing outside air to flow into the interior 113 of the dome 100 until the interior 113 of the dome 100 has a second set pressure different from the first set pressure. The magnitude of the negative pressure at the first set pressure may be, for example, -40 mmHg to -50 mmHg.

The first operation may be performed by respectively controlling the pump 410 and the control valve 430 as described above with reference to FIG. 11. In more detail, by stopping the operation of the pump 410 and opening the control valve 430, the outside air may be introduced into the dome and thus the negative pressure of the first set pressure may be released by a predetermined quantity.

Further, the breast volume measuring method may include a second operation of calculating the breast volume based on a flow rate of air introduced into the interior 113 of the dome 100 until the interior of the dome has the second set pressure. The magnitude of the negative pressure at the second set pressure may be, for example, -10 mmHg. That is, the magnitude of the negative pressure at the first set pressure may be greater than the magnitude of the negative pressure at the first set pressure.

Referring to FIG. 20, the second operation may include measuring a differential pressure between two different points 631 and 635 in an inlet passage 630 during a process of introducing outside air into the interior of the dome, determining a flow velocity from the differential pressure, calculating a flow rate by integrating the flow velocity with respect to time, and calculating a volume of the air introduced into the dome by integrating the flow rate with respect to time.

In summary, in a state in which the interior of the dome is maintained at the negative pressure of the first set pressure, the operation of the pump may be stopped, and the control valve 430 may be opened so that the outside air flows into the dome until an internal pressure of the dome reaches the negative pressure of the second set pressure, and the volume of the air may be calculated using the flow rate measured while the outside air flows into the dome.

In a Venturi tube structure shown in FIG. 20, a differential pressure between a first point (for example, the point of the reference numeral 631) and a second point (for example, the point of the reference numeral 635) that have passage sectional areas, which are sequentially decreased in an inflow direction of outside air, may be measured.

Equation 1 and Equation 2 below are obtained using the continuity equation and Bernoulli's equation.

[Equation 1]

$$Q = v_1 A_1 = v_2 A_2$$

[Equation 2]

$$P_1 - P_2 = \frac{\rho}{2}\left(v_2^2 - v_1^2\right)$$

In Equations 1 and 2, Q represents a flow rate, $P_1$ represents a pressure at the first point, P2 represents a pressure at the second point, $v_1$ represents a flow velocity at the first point, $v_2$ represents a flow velocity at the second point, $A_1$ represents a sectional area of a first position, and $A_2$ represents a sectional area of a second position.

When Equation 1 and Equation 2 are summarized as an equation concerning the flow rate Q, Equation 3 below is obtained.

[Equation 3]

$$Q = A_1 \sqrt{\frac{2(P_1 - P_2)}{\rho\left(\left(\frac{A_1}{A_2}\right)^2 - 1\right)}} = A_2 \sqrt{\frac{2(P_1 - P_2)}{\rho\left(1 - \left(\frac{A_2}{A_1}\right)^2\right)}}$$

When a flow rate value calculated through Equation 3 is multiplied by a flow coefficient C of the Venturi tube, Equation 4 below is obtained.

[Equation 4]

$$C_{venturi} = 0.9858 - 0.196 \times \left(\frac{D_{narrow}}{D_{Wide}}\right)^{4.5}$$

In Equation 4, $C_{venturi}$ represents the flow coefficient of the Venturi tube, $D_{narrow}$ represents a diameter of the first point, and $D_{wide}$ represents a diameter of the second point.

Further, the volume may be calculated using the sum of flow rates.

In the above Equation, a volume Vol may be calculated using the calculated sum Qt of flow rates and a change in pressure within the dome.

[Equation 5]

$$Q_t = \sum Q$$

[Equation 6]

$$Vol = \frac{Q_t}{P_{dome2} - P_{dome1}}$$

In Equation 6, $P_{dome1}$ represents the first set pressure and $P_{dome2}$ represents the second set pressure.

For example, an equation of calculating a volume using a cumulative sum of differential pressures (differences) may be implemented and used as a mathematical model, such as Equation 7 below, based on actually measured data.

[Equation 7]

$$V = P \times (-0.19086) - 522.81$$

In Equation 7, P represents only the number of a cumulative sum Pa of differential pressures measured in units of time of 50 ms, from which a unit is omitted, and V represents a resultant value of calculation of an expression at the right side of an equal sign, and Equation 7 is configured such that a resultant value V may be used as a volume by adding a volume unit cc thereto.

Further, the two different points 631 and 635 may have different sectional areas, in which air flows, in the inlet line 630.

Further, pressure sensors P1 and P2 may be provided at the two different points, respectively.

Further, in the determining of the flow velocity from the differential pressure, the flow velocity may be determined based on a differential pressure-flow velocity table which is prepared in advance using a flow velocity sensor.

Further, the magnitude of the negative pressure at the first set pressure may be greater than the magnitude of the negative pressure at the second set pressure, and the second set pressure may not have a zero.

Further, the breast volume measuring method may include performing the first operation and the second operation a plurality of times and measuring a change in the breast volume based on a first volume of air introduced into the dome that is calculated for a first time, and a second volume of air introduced into the dome that is calculated for a second time.

That is, depending on a wearer's usage state, the first air volume may be measured by performing the first operation and the second operation on a first day of use (for the first time), the second volume of air may be measured by performing the first operation and the second operation on a second day of use (for the second time) again, and the change in the breast volume may be measured by comparing the first air volume and the second volume of air .

Here, the breast volume measuring method may include determining that the breast volume has increased when the second volume of air measured for the second time is less than the first volume of air measured for the first time.

Further, the first operation may include generating negative pressure within the dome up to the first set pressure (the control valve is in a state of being closed) in a state in which the dome is worn on the wearer's breast, and stopping the operation of the pump when the interior of the dome has the first set pressure as described above with reference to FIG. 10.

In summary, the breast volume measuring method may include performing the first operation of stopping the operation of the pump when the interior of the dome has the first set pressure and calculating a volume of the air introduced into the dome by calculating a flow rate of the air introduced into the dome until the interior of the dome has the second set pressure different from the first set pressure while allowing outside air to flow into the dome, and an operation of repeatedly performing the first operation two times or more and measuring a change in breast volume based on a difference between the air volumes for the respective times.

The above breast volume measuring method may be configured to allow the control unit to measure a breast volume by enabling a differential pressure on the second flow line 702 of the above-described suction apparatus 300 to be measured. For example, the breast volume measuring method may be configured to measure a differential pressure in the discharge pipe 470 of the second flow line. In this case, two different points 631 and 635 of the discharge pipe 470 may have different sectional areas in which air flows, and pressure sensors P1 and P2 may be provided at the two different points 631 and 635, respectively.

In more detail, the tissue expansion apparatus having a breast volume measurement function may include the domes 100 for tissue expansion and the suction apparatus 300 generating negative pressure within the dome 100.

As described above, the suction apparatus 300 includes the pump 410 configured to generate negative pressure within the dome, the first flow line 701 connecting the interior of the dome to the inflow port of the pump, the second flow line 702 connecting the outflow port of the pump to the external atmosphere, the control valve 430 having the first port connected to the first flow line and the second port connected to the second flow line, the first pressure sensor P1 and the second pressure sensor P2 disposed at two different points in the line 630, through which outside air flows into the control valve 430, and the control unit 490 configured to control the pump and the control valve.

When the pump 410 operates and the control valve 430 is closed, as illustrated with reference to FIG. 10, the interior of the dome has the first set pressure, and when the operation of the pump 410 is stopped and the control valve 430 is opened, as illustrated with reference to FIG. 11, the outside air flows into the dome 100 and the interior of the dome has the second set pressure lower than the first set pressure.

Further, the control unit 490 is provided to perform a breast volume measurement mode to measure a change in breast volume.

Here, the control unit 490 may be provided, in the breast volume measurement mode, to calculate a volume of air introduced into the dome by stopping the operation of the pump and opening the control valve to allow outside air to flow into the dome when the interior of the dome has the first set pressure and then calculating a flow rate of the air introduced into the dome until the interior of the dome has the second set pressure different from the first set pressure, and when the air volume is measured a plurality of times, to measure a change in breast volume based on a difference between the air volumes measured for the respective times.

The control unit 490 may be provided to measure a differential pressure using the first pressure sensor P1 and the second pressure sensor P2, determine a flow velocity from the differential pressure, calculate a flow rate by integrating the flow velocity with respect to time, and calculate an air volume by integrating the flow rate with respect to time.

Further, as described above, the two different points may be points having different sectional areas, in which air flows, in the flow line.

Referring to FIG. 24, the control unit 490 may be provided to transmit breast volume change information to an external terminal 20. In more detail, the suction apparatus 300 may be provided to communicate with the external terminal 20 through wireless communication (for example, Wi-Fi, Bluetooth, or the like). The terminal 20 may include a smartphone, a laptop computer, a computer, or the like.

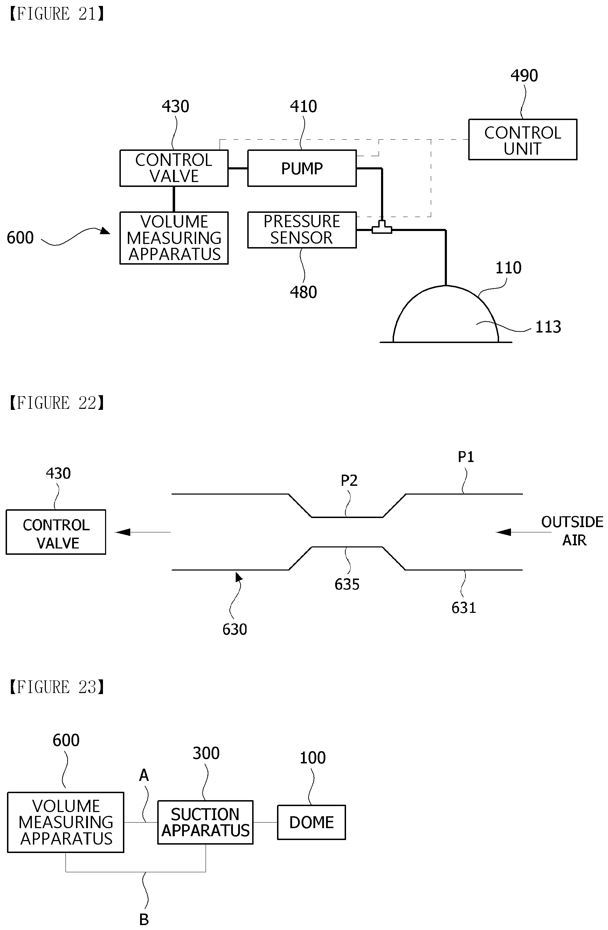

In contrast, as illustrated in FIGS. 21 to 23, a volume measuring apparatus 600 capable of measuring a differential pressure may be separately provided, and the volume measuring apparatus 600 may be configured to be detachably connected to the outlet 303 of the suction apparatus 300.

According to one embodiment of the present invention, the volume measuring apparatus 600 is an apparatus mounted on the tissue expansion apparatus 10 including the suction apparatus 300 configured to generate negative pressure within the dome, and includes a housing 601 having an inlet 610 through which outside air is introduced and an outlet 620 for supplying the outside air to the suction apparatus 300.

Further, the housing 601 may be detachably mounted on the suction apparatus 300 such that the outlet is connected to the suction apparatus 300 so that a fluid is movable therebetween.

In more detail, the housing 601 may be mounted on the side panel 340 of the suction apparatus 300 so that the outlet 620 is connected to the third exposure hole 343 of the side panel 340.

In such a structure, the outside air introduced into the inlet 610 of the volume measuring apparatus 600 is movable to the discharge pipe 470 through the outlet 303 of the suction apparatus 300.

Further, the volume measuring apparatus 600 may include a flow line 630 connecting the inlet 610 to the outlet 620 in the housing 601, first and second pressure sensors P1 and P2 provided at two different points 631 and 635 of the flow line 630, and a control unit 660 (also referred to as a "second control unit" in the following description) configured to output pressure information measured by the first and second pressure sensors P1 and P2 to the suction apparatus 300. Here, the pressure information may include a differential pressure between the two different points respectively measured by the first and second pressure sensors P1 and P2.

Further, the two different points may be points having different sectional areas, in which air flows, in the flow line 630.

Further, the volume measuring apparatus 600 may further include a power port 640 for supplying power to the pressure sensors. Here, the power port 640 may be provided to be connected to the suction apparatus 300, and specifically, to the connection terminal of the suction apparatus 300.

Further, the volume measuring apparatus 600 may further include a data port 670 for outputting the differential pressure data calculated by the control unit 660 to the suction apparatus 300, and the data port 670 may be provided to be connected to the suction apparatus 300.

For example, the power port 640 and the data port 670 may be formed as a single output terminal 680, and the output terminal 680 may be in a state of being pulled out to the outside of the housing 601 through a cable. The output terminal 680 may be provided to be connected to the above-described connection terminal of the suction apparatus.

That is, to perform the breast volume measurement mode, the volume measuring apparatus 600 is mountable to the suction apparatus 300 as necessary, thereby enabling miniaturization and slimming of the suction apparatus 300.

Further, the volume measuring apparatus 600 may receive power from the suction apparatus 300, measure a differential pressure alone, and transmit the measured differential pressure data to the suction apparatus 300 to allow the suction apparatus 300 to perform the breast volume measurement, and thus the breast volume apparatus 600 may be manufactured in a compact size.

Further, a tissue expansion apparatus 10 according to another embodiment of the present invention includes domes 100 worn on a wearer's body tissue, a suction apparatus 300 configured to generate negative pressure within the dome 100, and a volume measuring apparatus 600 mounted on the suction apparatus 300 to measure a volume of a body tissue (for example, a breast) received in the dome.

Hereinafter, a structure in which the functions of the suction apparatus 300 and the volume measuring apparatus 600 are separated will be described in detail.

The suction apparatus 300 includes a pump configured to generate negative pressure within the dome, a first flow line connecting an interior of the dome to an inflow port of the pump, a second flow line connecting an outflow port of the pump to the external atmosphere, a control valve having a first port connected to the first flow line and a second port connected to the second flow line, and a first control unit configured to control the pump and the control valve.

Further, the volume measuring apparatus includes an inlet configured to allow outside air to flow thereinto, an outlet configured to supply the outside air to the second flow line of the suction apparatus, a housing having the inlet and the outlet, a flow line connecting the inlet to the outlet in the housing, first and second pressure sensors provided at two different points of the flow line, and a second control unit configured to output a differential pressure measured by the first and second pressure sensors to the suction apparatus.

Here, when the pump operates and the control valve is closed, the interior of the dome has a first set pressure, and when the operation of the pump is stopped and the control valve is opened, the outside air flows into the dome through the volume measuring apparatus and the interior of the dome has a second set pressure lower than the first set pressure.

Further, a first control unit 490 is provided to perform a breast volume measurement mode for measuring a change in breast volume, and when the volume measuring apparatus is mounted on the suction apparatus, the first control unit is provided to, in the breast volume measurement mode, calculate a volume of air introduced into the dome by stopping the operation of the pump when the interior of the dome has the first set pressure and calculating a flow rate of the air introduced through the volume measuring apparatus until the interior of the dome has the second set pressure different from the first set pressure while allowing the outside air to flow into the dome, and, when the air volume is measured a plurality of times, measure a change in breast volume based on a difference between the air volumes measured for the respective times.

Further, the first control unit 490 is provided to determine a flow velocity from a differential pressure transmitted from a second control unit 660, calculate a flow rate by integrating the flow velocity with respect to time, and calculate an air volume by integrating the flow rate with respect to time. Further, the flow velocity may be determined based on a differential pressure-flow velocity table which is stored in advance.

Further, as described above, the two different points may be points having different sectional areas, in which air flows, in a discharge line.

Further, as described above, the volume measuring apparatus 600 may include an output terminal 680 electrically connectable to a battery 495 of the suction apparatus 300.

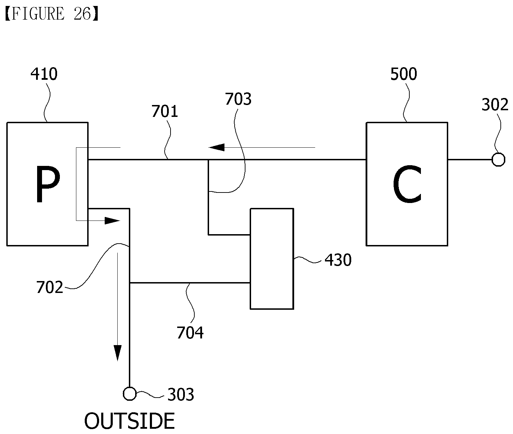

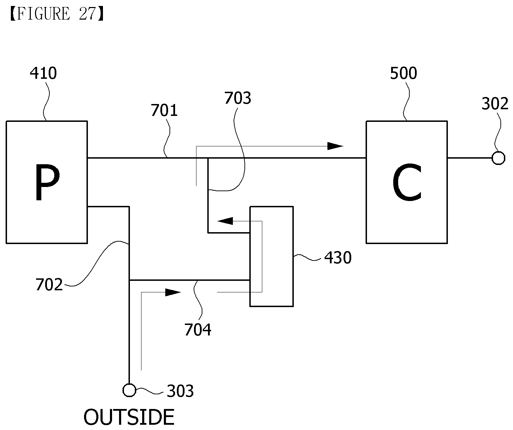

FIGS. 25 and 26 are separate perspective views of a canister 500 constituting a suction apparatus in accordance with one embodiment of the present invention, and FIG. 27 is a cross-sectional view of a state in which all elements shown in FIG. 25 are assembled.

In the present embodiment, a tissue expansion apparatus includes domes configured to receive a wearer's body tissue and a suction apparatus mounted on the dome to generate negative pressure within the dome.

Here, a suction apparatus 300 includes a housing 301 provided with an inlet 302 and an outlet 303 connected to an interior of the dome, a pump 410 disposed in the housing 301 and configured to suction air in the dome through the inlet, and a canister 600 mounted at the inlet 302 of the housing 301.

The canister 600 includes a suctioning port connected to the interior of the dome, a discharge port connected to the inlet, and a flow path F connecting the suctioning port to the discharge port.

Referring to FIG. 27, the flow path F includes a plurality of first sections prepared such that air flows parallel to a central axis direction of the suctioning port and second sections prepared to connect two neighboring first sections such that air flows in a radial direction based on a central axis of the suctioning port.

Further, the second sections may be prepared such that air flows in a certain rotating direction based on the central axis of the suctioning port.

Further, the suctioning port and the discharge port of the canister may be prepared to be located coaxially.

In more detail, the canister 600 includes a suction member 510 formed of an elastic member and forming the suctioning port and a lower case 520 on which the suction member 510 is mounted. The canister 600 includes an upper case 550 provided with the discharge port and mounted on the lower case 520 to form a predetermined flow space and a partition member 530 disposed in the flow space and configured to divide the flow space into the plurality of first sections and second sections. Further, the canister 600 includes a filter 540 disposed between the partition member 530 and the upper case 550.

As described above with reference to FIG. 1, unlike the structure in which the dome and the suction apparatus are spaced far away from each other through the connection tube, when the suction apparatus is detachably mounted on the dome, a flow path connecting the interior of the dome to the suction apparatus becomes shortened, and thus, moisture may be generated.

However, as in the canister 600 according to the present embodiment, when the flow path F is formed, moisture may be prevented from being generated.

In more detail, the lower case 520 may include a plurality of first flow guides 521, which have a ring shape and different diameters and are arranged concentrically, on a first surface thereof facing the upper case 530. Further, the partition member 530 may include a second flow guide 531, which is located between two neighboring first flow guides 521, on a first surface thereof facing the lower case.

Here, the first flow guide may have a height in which the first flow guide does not come into contact with the partition member, and the second flow guide may have a height in which the second flow guide does not come into contact with the lower case.

In such a structure, a space between the first flow guide 521 and the second flow guide 531 may form the first section, and a space between the second flow guide 531 and the first surface of the lower case 520 may form the second section.

Further, a plurality of spacers 532 and 533 may be provided on a second surface of the partition member 530, which is a surface opposite to the first surface, to support the filter. The plurality of spacers 532 and 533 may be arranged to be spaced apart from each other at predetermined intervals in radial and circumferential directions.

A space between the second surface of the partition member 530 and the filter 540 is connected to the discharge port of the upper case 550.

Further, in order to absorb moisture, super absorbent polymer (SAP) particles may be disposed in the flow space.

Further, the canister 600 may include a sealing cap 560 mounted at the discharge port of the upper case 550 and coming into contact with the inlet of the housing 301 of the suction apparatus 300.

Here, an inflow guide 316 having a ring shape is provided on a bottom surface of the main case 310 of the suction apparatus 300, the discharge port of the upper case 550 is disposed in the inflow guide 316, and the sealing cap 560 is closely adhered to an inner circumferential surface of the inflow guide 316.

Further, the outflow port of the upper case 550 is connected to a flow path of the negative pressure sensing unit 380 such that a fluid is movable therebetween.

The exemplary embodiments of the present invention described above have been disclosed for the purpose of illustration, and various changes, modifications, and additions may be made within the spirit and scope of the invention by those skilled in the art. These modifications, changes, and additions are to be regarded as belonging to the scope of the claims of the present invention.

[Industrial Applicability]

A dome for tissue expansion, a canister, a suction apparatus, and a tissue expansion apparatus including the same in accordance with one embodiment of the present invention have the following applicability.

By providing a structure in which a suction apparatus is detachably mounted on a dome, a wearer's convenience in use may be improved, and since the suction apparatus is mounted on the dome, it is possible to shorten the physical distance between a negative pressure source and a negative pressure generation space.

Further, moisture may be prevented from being generated in a canister in a suction apparatus during operation.

**Claims**

1. A suction apparatus comprising:

   a housing having an inlet and an outlet;
   a pump disposed in the housing and configured to suction air introduced through the inlet;
   a first flow line connecting the inlet to an inflow port of the pump;
   a second flow line connecting an outflow port of the pump to the external atmosphere;
   a control valve disposed in the housing and having a first port connected to the first flow line and a second port connected to the second flow line;
   a battery disposed in the housing and configured to supply power to the pump; and
   a control unit configured to control each of the pump and the control valve.

2. The suction apparatus of claim 1, further comprising an air distribution member mounted on the pump and having an inlet passage forming at least a portion of the first flow line and connected to the inflow port of the pump and an outlet passage forming at least a portion of the second flow line and connected to the outflow port of the pump.

3. The suction apparatus of claim 2, wherein the control valve is mounted on the air distribution member such that the first port is connected to the inlet passage and the second port is connected to the outlet passage.

4. The suction apparatus of claim 3, wherein, when the pump operates and the control valve is closed, air is suctioned into the pump along the inlet passage of the air distribution member through the inlet, and the air is discharged to the outside along the outlet passage.

5. The suction apparatus of claim 4, wherein when the pump is stopped and the control valve is open, outside air flows into the second port of the control valve along the outlet passage, passes through the first port, and is movable to the inlet along the inlet passage.

6. The suction apparatus of claim 2, wherein
   a flow hole is provided in the inlet passage of the air distribution member, and
   a check valve, for allowing a flow only in a direction toward the inflow port of the pump, is provided at the flow hole.

7. The suction apparatus of claim 6, wherein the check valve opens the inlet passage toward the inflow port of the pump when the pump operates and closes the inlet passage when the pump is stopped.

8. The suction apparatus of claim 6, wherein the check valve includes:

   a shaft inserted into the flow hole;
   a sealing member mounted on the shaft and coming into contact with the flow hole; and
   an elastic member provided to be compressed when the pump operates and thus the shaft moves toward the pump,
   wherein, when the shaft moves toward the pump, the sealing member is separated from the flow hole.

9. The suction apparatus of claim 1, further comprising a discharge pipe connecting an outlet passage of an air distribution member to the outlet of the housing and forming at least a portion of the second flow line.

10. The suction apparatus of claim 1, further comprising a negative pressure sensing unit which is disposed in the housing, has a flow path connecting the inlet to an inlet passage of an air distribution member, and is configured to measure a pressure of the air suctioned through the inlet.

**11.** A tissue expansion apparatus comprising:

a dome for tissue expansion; and
a suction apparatus detachably mounted to the dome,

wherein the dome includes:

a cap including a receiving part covering a partial region of a wearer's body, a suction port connected to the receiving part such that a fluid is movable therebetween, and a mounting part surrounding the suction port; and
a contact part provided along an edge of the cap to come into contact with the wearer's body, and

the suction apparatus includes:

a housing that has an inlet connected to the suction port and an outlet, and is accommodated in the mounting part;
a pump disposed in the housing and configured to suction air introduced through the inlet;
a first flow line connecting the inlet to an inflow port of the pump;
a second flow line connecting an outflow port of the pump to the external atmosphere;
a control valve disposed in the housing and having a first port connected to the first flow line and a second port connected to the second flow line;
a battery disposed in the housing and configured to supply power to the pump; and
a control unit configured to control each of the pump and the control valve.

**12.** The tissue expansion apparatus of claim 11, wherein at least one magnet is provided in each of the mounting part of the cap and the housing.

**13.** The tissue expansion apparatus of claim 11, further comprising a canister mounted on a side of the inlet of the housing, wherein the canister includes a suctioning port connected to an interior of the dome, a discharge port connected to the inlet, and a flow path connecting the suctioning port to the discharge port, and
the flow path includes a plurality of first sections provided such that air flows in parallel to a central axis direction of the suctioning port and second sections prepared to connect two neighboring first sections such that air flows in a radial direction based on a central axis of the suctioning port.

**14.** The tissue expansion apparatus of claim 13, wherein the second sections are prepared such that air flows in a certain rotating direction based on the central axis of the suctioning port.

**15.** The tissue expansion apparatus of claim 14, wherein the suctioning port and the discharge port are prepared to be located coaxially.

**16.** The tissue expansion apparatus of claim 13, wherein the canister includes:

a suction member formed of an elastic member and forming the suctioning port;
a lower case on which the suction member is mounted;
an upper case provided with the discharge port and mounted on the lower case to form a predetermined flow space;
a partition member disposed in the flow space and configured to divide the flow space into the plurality of first sections and second sections; and
a filter disposed between the partition member and the upper case.

**17.** The tissue expansion apparatus of claim 16, wherein
the lower case includes a plurality of first flow guides, which have a ring shape and different diameters and are arranged concentrically, on a first surface thereof facing the upper case,
the upper case includes a second flow guide, which is located between two neighboring first flow guides, on a first surface thereof facing the upper case, and
the first flow guide has a height in which the first flow guide does not come into contact with the upper case, and the second flow guide has a height in which the second flow guide does not come into contact with the lower case.

**18.** The tissue expansion apparatus of claim 17, wherein
a space between the first flow guide and the second flow guide forms the first section, and
a space between the second flow guide and the first surface of the lower case forms the second section.

**19.** The tissue expansion apparatus of claim 17, wherein a plurality of spacers are provided on a second surface of the upper case, which is a surface opposite to the first surface, to support the filter.

**20.** The tissue expansion apparatus of claim 17, wherein a space between a second surface of the upper case and the filter is connected to the discharge port.

【FIGURE 1】

【FIGURE 2】

【FIGURE 3】

I      111      O

100-1

122

t2

124

t1

121

123

【FIGURE 4】

I          O

121

100-1

111

122      124

w2      w1

123

EP 3 735 952 A1

【FIGURE 5】

20

【FIGURE 6】

【FIGURE 7】

【FIGURE 8】

【FIGURE 9】

【FIGURE 10】

【FIGURE 11】

300

301

360

500

【FIGURE 12】

500

560

550

540

532

533

530

521

520

510

【FIGURE 13】

500

560

550

540

530

531

520

510

【FIGURE 14】

【FIGURE 15】

【FIGURE 16】

【FIGURE 17】

【FIGURE 18】

【FIGURE 19】

【FIGURE 20】

【FIGURE 21】

490
CONTROL UNIT

430
CONTROL VALVE

410
PUMP

600
VOLUME MEASURING APPARATUS

PRESSURE SENSOR

480

110

113

【FIGURE 22】

430
CONTROL VALVE

P2

P1

OUTSIDE AIR

635

631

630

【FIGURE 23】

600
VOLUME MEASURING APPARATUS

A

300
SUCTION APPARATUS

100
DOME

B

【FIGURE 24】

【FIGURE 25】

【FIGURE 26】

410
500
302
701
703
P
C
702
430
704
303
OUTSIDE

【FIGURE 27】

## INTERNATIONAL SEARCH REPORT

| International application No. |
| --- |
| **PCT/KR2018/016729** |

### A. CLASSIFICATION OF SUBJECT MATTER

*A61H 9/00(2006.01)i, A61H 19/00(2006.01)i*

According to International Patent Classification (IPC) or to both national classification and IPC

### B. FIELDS SEARCHED

Minimum documentation searched (classification system followed by classification symbols)
A61H 9/00; A61F 5/00; A61F 5/03; A61H 7/00; A61M 29/00; A61H 19/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: aspirator, tissue expansion, housing, pump, flow line, control valve, battery, dome

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2014-0045727 A (KIM, Kwang Ryel) 17 April 2014<br>See claim 1; paragraphs [0031]-[0046]; figure 1. | 1-20 |
| A | KR 10-1703259 B1 (CLASSYS INC.) 06 February 2017<br>See the entire document. | 1-20 |
| A | KR 20-1998-0055645 U (CHO, Young Ok) 07 October 1998<br>See the entire document. | 1-20 |
| A | JP 11-514892 A (KHOURI BIOMEDICAL RESEARCH, INC.) 21 December 1999<br>See the entire document. | 1-20 |
| A | KR 10-1716127 B1 (CG BIO CO., LTD.) 15 March 2017<br>See the entire document. | 1-20 |

| ☐ Further documents are listed in the continuation of Box C. | ☒ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 10 APRIL 2019 (10.04.2019) | **10 APRIL 2019 (10.04.2019)** |

| Name and mailing address of the ISA/KR | Authorized officer |
| --- | --- |
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

## INTERNATIONAL SEARCH REPORT
Information on patent family members

| | International application No. |
|---|---|
| | **PCT/KR2018/016729** |

| Patent document cited in search report | Publication date | Patent family member | Publication date |
|---|---|---|---|
| KR 10-2014-0045727 A | 17/04/2014 | None | |
| KR 10-1703259 B1 | 06/02/2017 | None | |
| KR 20-1998-0055645 U | 07/10/1998 | KR 20-0207684 Y1 | 15/01/2001 |
| JP 11-514892 A | 21/12/1999 | AT 235866 T | 15/04/2003 |
| | | AT 241322 T | 15/06/2003 |
| | | AU 2188295 A | 23/10/1995 |
| | | AU 6899496 A | 12/03/1997 |
| | | AU 705754 B2 | 03/06/1999 |
| | | AU 706441 B2 | 17/06/1999 |
| | | CA 2186725 A1 | 12/10/1995 |
| | | CA 2186725 C | 26/07/2005 |
| | | CA 2229992 A1 | 27/02/1997 |
| | | CA 2229992 C | 03/08/2010 |
| | | DE 69530896 T2 | 19/05/2004 |
| | | DE 69627159 T2 | 12/02/2004 |
| | | EP 0708620 A1 | 04/09/2002 |
| | | EP 0708620 A4 | 05/03/1997 |
| | | EP 0708620 B1 | 28/05/2003 |
| | | EP 0844857 A1 | 04/09/2002 |
| | | EP 0844857 A4 | 17/11/1999 |
| | | EP 0844857 B1 | 02/04/2003 |
| | | ES 2194112 T3 | 16/11/2003 |
| | | ES 2199988 T3 | 01/03/2004 |
| | | JP 10-500037 A | 06/01/1998 |
| | | JP 3501459 B2 | 02/03/2004 |
| | | JP 3501463 B2 | 02/03/2004 |
| | | US 2001-0031911 A1 | 18/10/2001 |
| | | US 2005-0245850 A1 | 03/11/2005 |
| | | US 5536233 A | 16/07/1996 |
| | | US 5662583 A | 02/09/1997 |
| | | US 5676634 A | 14/10/1997 |
| | | US 5695445 A | 09/12/1997 |
| | | US 5701917 A | 30/12/1997 |
| | | US 6500112 B1 | 31/12/2002 |
| | | WO 2006-110327 A2 | 19/10/2006 |
| | | WO 2006-110327 A3 | 15/11/2007 |
| | | WO 95-26690 A1 | 12/10/1995 |
| | | WO 97-06756 A1 | 27/02/1997 |
| KR 10-1716127 B1 | 15/03/2017 | KR 10-2016-0074146 A | 28/06/2016 |
| | | KR 10-2016-0105758 A | 07/09/2016 |

Form PCT/ISA/210 (patent family annex) (January 2015)